# EUROPEAN PATENT SPECIFICATION

(11) **EP 1 162 987 B1**
(45) Date of publication and mention of the grant of the patent: **20.11.2002**
(21) Application number: 00914133.4
(22) Date of filing: 15.03.2000
(51) Int. Cl.: A61K 35/74, A61P 1/12

(54) **PHARMACEUTICAL COMPOSITION FOR MEDICAL AND VETERINARY USE FOR REGENERATING INTESTINAL FLORA IN DIARRHOEA OR DYSPEPTIC SYNDROME**
PHARMAZEUTISCHE ZUSAMMENSETZUNGEN FÜR MEDIZINISCHE UND VETERINÄRE VERWENDUNG ZUR VERBESSERUNG DER DARMFLORA BEI DYSPEPTISCHEM SYNDROM UND DIARRHOEEN AUF DEM VETERINÄRSEKTOR
COMPOSITION PHARMACEUTIQUE A USAGE MEDICAL ET VETERINAIRE UTILISEE POUR REGENERER LA FLORE INTESTINALE EN CAS DE DIARRHEE OU DE SYNDROME DYSPEPTIQUE

(30) Priority: 15.03.1999 IT FI990051
(43) Date of publication of application: 19.12.2001
(73) Proprietor: Italmed S.N.C. Di Galli G. E Pacini G., 50132 Firenze (IT)
(72) Inventor: GALLI, Giovanna, I-50100 Firenze (IT)
(74) Representative: Celestino, Marco
(86) International application number: EP0002271
(87) International publication number: WO00054788

(56) References cited:
- EP-A- 0 426 463
- WO-A-93/00012
- WO-A-97/07822
- FR-A- 2 674 755

## Description

### Field of the invention

The present invention relates to a pharmaceutical composition for medical and veterinary use, and precisely for a so called "symbiotic" use, based on living lactic acid bacteria and not adsorbable carbohydrates, used for the regeneration of the gut microbial eubiosis in the diarrhoic syndrome of infectious origin or in that associated to the administration of antibiotics.

### Description of the prior art

At the birth the digestive tube of a human or of other mammal subjects is sterile; animals raised in sterile environment do not mature micro-organisms in the gut. This fact evidences that the intestinal flora comes exclusively from the outer environment.

It is known that the bacteria of the intestinal flora colonise the digestive system of the new-born according to an oral-anal direction. In a wide range of animals, among which also humans, coliforms and streptococci establish in the digestive tube within a few hours from the birth . Twenty four hours later anaerobic lactobacilli and the enterococci become stable and grow in number gradually during the successive ten-twenty days, whereas the coliforms decrease dramatically. Bacterioids, which will form dominant part of the colon flora, for the first time appear after ten days and quickly grow in number in the next two weeks. Approximately three or four weeks after birth the characteristic intestinal flora is well defined and will not change substantially thereafter.

The type and the number of micro-organisms that normally are provided in the various portions of the digestive tube vary significantly, and also the pH varies according to the gastro-intestinal portion considered. The stomach and the small intestine contain normally a relatively low number of bacteria. In fact culture from material coming from the jejunum do not show any bacterial growth in about a third of sound subjects that have been tested. When micro-organisms are provided they usually consist of lactobacilli and enterococci, gram-positive aerobic or facultative anaerobic detected in concentration up to 10³ for gram of material in the stomach, and up to 10⁴ for each gram in the jejunum . The average pH is 1,5 in the stomach and varies from 6 to 7 in the jejunum. Coliforms can be present temporarily in the jejunum of a sound individual, but seldom exceeding 10³ for gram of material and probably they are contaminatedng organisms that are being conveyed towards the colon. The anaerobic bacterioids are not present in the proximal part of the gut of a sound individual.

Microbiologically the ileum is a transition zone between the sparse organisms population of the stomach and in the proximal intestine, and the dense population of anaerobic bacteria of the colon. In the ileum the concentration of micro-organisms increases up to 10⁵-10⁹ organisms for gram of material. Enterobacteria, including the coliforms, are regularly present quite largely in the ileum. The purely anaerobic bacteria, that cannot live normally in the jejunum, colonise frequently the ileum. The average pH of this region of the gut is about 7.5.

The most relevant change in the intestinal flora occurs through the ileum-caecal valve. In fact the number of micro-organisms increases up to a million times, and approximately up to 10⁹-10¹² micro-organisms for gram of material in the colon. The pH in the caecum is about to 6.8-7.3.

The intestinal flora of the large intestine is dominated by the anaerobic micro-organisms such as bacterioids, bifidobacteria, anaerobic lactobacilli and clostridium bacteria. The complexity of the intestinal flora in the colon is evidenced by more than 400 different strains in a single subject detected after bacteriologic analysis.

The factors that limit the bacterial growth are essentially three, i.e. gastric acidity, intestinal motility, interaction among bacteria.

Firstly, achloridria, i.e. lack of hydrochloric acid in the gastric juice, owing to atrophy of the gastric mucous membrane, or to a gastric resection, allows an increase of the number of micro-organisms that reach the small intestine.

A second relevant factor that limits the bacterial proliferation in the small intestine is definitely the cleaning action owing to peristalsis that causes the quick elimination of the micro-organisms. The mucus, present on the gut mucous membrane, aids this mechanical process of removing bacteria, since it is known that bacteria tend to concentrate on the mucus layer that adheres to the gut mucous membrane.

In the large intestine, where the material remains for a longer time, the bacterial growth is very high. As known from literature even if most of micro-organisms are killed by the acid contained in the stomach, the bacteria that survive reach the small intestine and are viable also in the colon that they reach in a relatively short time. In vitro models representing the conditions of the gastric, duodenal, jejunal and ileal intestine have revealed the rate of survival of different strains of lactobacilli by means of a dynamic computer technique. The rate of survival of lactobacilli that reach the ileum, is about 5-10%, whereas this rate can rise to a maximum of 30% for bifidobacteria. On the other hand, for some strains such as streptococcus thermophilus the rate of survival is very low (about 1%). These data have been confirmed by researches on voluntary sound subjects using marked bacteria. It is then apparent the high reduction (from 70 to 99%) of the population of lactic acid bacteria that are administered orally as therapy or as prophylaxis. This "physiologic sterilisation" occurs mainly in the stomach owing to the strongly acid environment, but also in the small intestine owing to the biliary acids .

The third mechanism that controls the bacterial growth in the gut is the interaction that the bacteria have with one another. These interactions are complex and numerous, and are carried out by the lactic acid bacteria against pathogen bacteria, formed for example after antibiotic therapy. Among the most relevant interaction there are:
A) Mutual competition for the available nutrients
B) Modification of the intraluminal pH
C) Competition for sticking onto the gastro-enteric mucous membrane (without sticking the bacteria are quickly flushed away from the gastro-enteric tube).
D) endogenous production by the bacteria same of bacteriocines, products with high molecular weight that have antibacteric activity .
E) production of short chain fatty acids (acetic, propionic, butyric acids) that have bacteriostatic activity .
F) Deconjugation of the biliary acids, which become then inhibitors for some strains

Various types of diarrhoea are known: the "secretive diarrhoea " is caused by bacteric toxins or virus that irritate the intestinal mucous membrane thus determining an increase of both the water secretion and of electrolytes by the gut epithelium and an increase of peristalsis . Enteritis of this type is often the consequence of eating contaminated water or food. An example that can be included in this type of diarrhoea is the so called "diarrhoea of the traveller ".

The " osmotic diarrhoea " is the consequence of a low absorption of osmotically active solutes, whose presence in the gut cause an increase of fluids in the gut lumen. The intolerance to lactose is a classic example of osmotic diarrhoea . Are known also other types of diarrhoea said "exudative", characterised by the production of exudate owing to a damage of the mucous membrane (induced by radiation , ulcerous colitis, Crohn disease, etc.).

It is known furthermore that an antibiotic treatment can cause diarrhoea since it modifies the intestinal flora. During the use of antibiotics, in fact, there is a reduction of the antibiotic-sensitive bacteria, such as bifidobacteria and lactobacilli, and a simultaneous growth of antibiotic-resistant bacteria such as enterococci and coliforms that can produce a variety of syntomi among which diarrhoea.

Saprofyite bacteria that make up the intestinal flora, and in particular lactobacilli and bifidobacteria, cause carbohydrates not adsorbed in the small intestine to ferment and to turn into short chain acids, (acetic, propionic, butyric), which are quickly adsorbed passively through the intestinal wall. Such acids produced by the bacterial fermentation are essential for maintaining the integrity of the mucous membrane of the colon.

The etiopathogenetic essential moment of antibiotic-induced diarrhoea is the decrease of this process of fermentation that brings to a decrease of the production of these short chain acids and then to an intraluminar increase of the osmolar concentration, and above all to a lack of stimulation of the mucous membrane of the colon to adsorb water and salts. This phenomena is induced by the above cited short chain fatty acids. Moreover, one of the activity of the propionic, acetic, butyric acids is to inhibit the colon motility, and then a deficiency thereof increases peristalsis and then diarrhoea.

The reduction of micro- "protective" organisms(for example induced by antibiotics) allows the colonisation of pathogen organisms that can induce synptoms of different entity, starting for example from a not complex diarrhoea up to pseudomembranose colitis that causes the death of the patient. This severe of gut infection is caused to the production of a toxin delivered by Clostridium Difficile.

It is then apparent that for probiotics, i.e. a live microbial feed supplement which beneficially affects the host animal by improving its intestinal microbial balance, both present in food (such as yoghurt, fermented milks) and as food integrators (present on the market in tiny bottles, capsules, packets etc.), there is the problem of a high reduction of their activity, which is induced by the lactic acid bacteria, when they reach the colon owing to the low gastric pH and the biliary acids that they met through the stomach and small intestine.

Also for "pre-biotics', there is a partial reduction of product that reaches the colon, where they are then induced to fermentation by bacteria. "Pre-biotics' are substances that are not assimilated and adsorbed in the small intestine and that are beneficial since they selectively stimulate the growth and/or the activity of bacteria already present in the colon (like not adsorbable carbohydrates such as oligosaccharides among which lactulosium, lactitol, fruit oligosaccharides, the sorbitol, the mannitol, etc.). In particular, fruit oligosaccharides are partially hydrolised in the stomach (circa the 10-15%) and this amount is adsorbed in the small intestine.

Therefore the existing pharmaceutical composition based on not adsorbable carbohydrates and/or lactic acid bacteria still have various problems and are not totally acceptable.

### Summary of the invention

It is an object of the present invention a pharmaceutical composition capable of providing lactic acid bacteria and of protecting them in the stomach and in the small intestine against the action respectively of the gastric juice and of the biliary acids .

It is a particular object of the present invention a pharmaceutical composition capable of providing lactic acid bacteria and not adsorbable carbohydrates and of protecting the latter from the hydrolysis in the stomach by the gastric juice.

It is another object of the present invention a pharmaceutical composition capable of modulating the above protection so that pH in the stomach is rapidly increased, and at the same time of assuring a duration of this effect up to the complete emptying of the stomach after the consumption of the product (about 90 minutes).

It is still another object of the present invention a pharmaceutical composition capable of providing such a "protection system" which is bio-compatible with the living lactic acid bacteria, has a good organoleptic and a low industrial cost.

These and other objects are achieved by the pharmaceutical composition according to the invention, whose characteristic is that it comprises a calcium salt and an aluminium salt in addition to lactic acid bacteria and not adsorbable carbohydrates.

The association of a calcium salt and of an aluminium salt to lactic acid bacteria and not adsorbable carbohydrates, accomplishes in particular:
- sensible increasing of the units of lactic acid bacteria that pass the "stomach barrier" and reach the colon. The pH increases in the stomach to about 3.5-4 and allows an ideal habitat for the lactic acid bacteria same. This effect is obtained through the combined and modulated action of the calcium salt and of the aluminium salt. In fact whereas the former allows a quick pH rise, the latter maintains the achieved value of pH for a sufficient time (about 90 minutes) up a complete stomach emptying. For this reason, there is a substantial increase of living bacteria that pass the "stomach barrier" and then reach the colon, with amplification of the above described mechanisms of competition against pathogen bacteria.
- Minor loss of units of living lactic acid bacteria through small intestine. In fact the presence in small intestine of calcium salts and aluminium salts salifies the biliary acids, (that kill lactic acid bacteria) carrying then out, even if with a different mechanism, a further protection of the lactic-acid bacteria.
- Reduction of the hydrolysis of the not adsorbable carbohydrates in the stomach. Thus, the almost complete availability in the colon of such substances is obtained for being used by lactobacilli and bifidobacteria with a more effective and quick regeneration of the amount of short chained fatty acids (acetic, butyric, propionic) in the colon. The presence in gut of such substances, as above explained, is in fact essential against diarrhoea, since it induces the absorption of water and solutes, it reduces the gut peristalsis, and has a bacteriostatic activity (in particular acetic acid is produced mainly by bifidobacteria) opposing to the reproduction of the pathogen bacteria.
- Synergic effect in limiting diarrhoea of the aluminium and calcium salts and the action in the colon of the lactic acid bacteria, owing to the salifying action of the biliary acids . In fact, biliary acids, if not adsorbed by the small intestine, reach the colon and increase diarrhoea, since in the sound individual the biliary acids that reach the colon have a relevant task in stimulating defecation by means of the secretion of water and electrolytes).
- Lower cito-toxicity of the biliary acids on the mucous membrane since these are precipitated into calcium and aluminium salts. In fact it is well known the exposure of intestinal mucous membrane to deterioration and damages caused by biliary acids in this pathologic condition.
- The use of a calcium salt together with the lactic acid bacteria allows a significant increase, in the colon, of the fermentation of not adsorbable carbohydrates, thus further stimulating the growth of equal lactic acid bacteria and protein synthesis.
- The use of a calcium salt has an effect against diarrhoea owing to a direct inhibition of the secretion mechanisms and of gut motility.
- Calcium salts and aluminium salts do not affect the organoleptic of the final product and their implementation is not expensive.
- Reduction of the amount of lactic acid bacteria that have to be present in the daily administration, with respect to the average daily amount provided through other products present on the market. This allows a less expensive daily therapy.

In a clinical research patients have been treated with a product based on lactic acid bacteria and fruit oligosaccharides together with a calcium salt and -an aluminium salt, for preventing diarrhoea associated to antibiotic treatment (cefixime). This research has surprisingly revealed efficacy higher than equal products without the above described salts, in particular with respect to a product containing only lactic acid bacteria and with respect to a placebo. In this research, carried out in double caecum on 115 patients (60 men and 55 women) of age set between 18 and 59 years, in the prevention of the diarrhoea associated to antibiotic treatment with cefixime, four groups of patients have been compared:
A) 32 patients treated, twice a day, with lactic acid bacteria (100 billion of units), fruit oligosaccharides (3g), sodic aluminium silicate (3g), calcium carbonate (1g).
B) 27 patients treated, twice a day, with lactic acid bacteria (100 billion of units), fruit oligosaccharides (3g).
C) 31 patients treated, twice a day, with lactic acid bacteria (100 billion of units).
D) 29 patients treated, twice a day, with a placebo

The results of the research have been:

| Group | N° patients | treatment duration | Freq. diarrhoea |
|---|---|---|---|
| A | 32 | 10 days | 0% |
| B | 27 | 10 days | 7,4% |
| C | 31 | 10 days | 9,6% |
| D | 29 | 10 days | 18,7% |

According to the present invention the preferred calcium salt to obtain the desired effects is- calcium carbonate (between 500mg and 2500 mg), whereas the preferred aluminium salt is sodic aluminium silicate (between 500mg and 4500mg) The probiotic agents used for the present invention are lactic acid bacteria, in particular:
- bifidobacteria (between 1 billion and 100 billion of units); particularly preferred are bifidobacterium longum, bifidobacterium bifidum, bifidobacterium adolescentis, bifidobacterium breve and bifidobacterium infantis.
- lactobacilli (between 4 and 100 billion of units); particularly preferred are lactobacillus lactis, lactobacillus acidophilus, lactobacillus bulgaricus, lactobacillus helveticus, lactobacillus casei, lactobacillus plantarum, lactobacillus paracasei;
- streptococcus thermophilus (between 1 and 300 billion).

Among the many "bifidogenetic" agents (pre-biotics) for a composition according to the invention not adsorbable carbohydrates are used, and in particular the fruit oligosaccharides (between 1000mg and 5000mg) are preferred and the vitamins of group B and in particular Vit. B12 (from 0,5µg to 2µg), the Vit. B6 (from 0,5mg to 2mg), the pantothenic acid (from 2mg to 7mg).

The pharmaceutical composition object of the present invention comprises usual wetting agents (palmitate saccharose), fluidificating agents (colloidal silica), suspending agents (cellulose, carboxyimethylcellulose, sodium carboxyimethylcellulose), organoleptic correctors (orange flavour, lemon flavour, milk flavour).

A mixture of the above described components is then packaged into small packets.

Not limitative examples of the present invention are the following:

### EXAMPLE 1

A composition to medical use for regeneration of the intestinal microbial eubiosis in the diarrhoic and dyspeptic syndrome (colitis, diarrhoea, aspecific enteritis), in the prophylaxis and treatment of the intestinal dysmicrobism after antibiotics, with the following weight proportions:

| | |
|---|---|
| Calcium carbonate | 1000mg |
| Sodic aluminium silicate | 3000mg |
| Bifidobacterium longum | 5 billion of units |
| Lactobacillus lactis | 5 billion of units |
| Lactobacillus acidophilus | 20 billion of units |
| Lactobacillus casei | 20billion of units |
| Lactobacillus bulgaricus | 25 billion of units |
| Streptococcus thermophilus | 25 billion of units |
| Fruit oligosaccharides | 3000mg |
| Vit. B12 | 0,7µg |
| Vit. B6 | 0,7mg |
| Pantothenic acid | 2mg |
| Colloidal silica | 130mg |
| Microcristalline cellulose + CMC | 200mg |
| Palmitate saccharose | 120mg |
| Flavour (orange/lemon/milk) | 100mg |
| Deionised water (humidity) | q.b. to 8.000 mg |

It is used in the following way to control physiologically the intestinal flora: in the adult have a packet twice a day far from meals; In the children have a packet once a day far from meals. In the new born have half packet, once a day, mixed with milk. The average duration of the treatment is 1-2 weeks.

### EXAMPLE 2

A composition such as for example 1, with the same use indication and the following weight proportions:

| | |
|---|---|
| Calcium carbonate | 1000mg |
| Sodic aluminium silicate | 3000mg |
| Bifidobacterium bifidum | 20 billion of units |
| Lactobacillus paracasei | 20 billion of units |
| Tagatose | 2000mg |
| Fruit oligosaccharides | 3000mg |
| Vit. B12 | 0,7µg |
| Vit. B6 | 0,7mg |
| Pantothenic acid | 2mg |
| Colloidal silica | 130mg |
| Microcristalline cellulose + CMC | 200mg |
| Palmitate saccharose | 120mg |
| Flavour (orange/lemon/milk) | 100mg |
| Deionised water (humidity) | q.b. to 10.000 mg |

### EXAMPLE 3

A composition for veterinary use, for example for cats, dogs, cattle and pigs.

The composition is mixed to cereal and is provided in the form of pellets In particular, the pellets are obtained starting from a starch based material, filled or coated with a support that that has the weight proportions of calcium and aluminium salts and of bacteria such as for examples 1 or 2.

The excipients and the flavours can be chosen as a function of the animals to which they are to be administered.

## Claims

1. A pharmaceutical composition for medical and veterinary use for the regenerating intestinal flora in diarrhoea or dyspeptic syndrome (colitis, diarrhoea, aspecific enteritis), for prophylaxis and treatment of intestinal dismicrobism after use of antibiotics, for digestive diseases of the newborn artificially fed, for digestive diseases of farm animals and pets, containing living lactic acid bacteria, **characterised in that** it comprises an added calcium salt and an added aluminium salt.

2. A pharmaceutical composition according to claim 1, wherein the calcium salts used are chosen between: calcium carbonate, dibasic calcium phosphate, threebasic calcium phosphate or a mixture thereof

3. A pharmaceutical composition according to claim 1 wherein the aluminium salts used are chosen between: sodic aluminium silicate, calcic aluminium silicate, aluminium magnesium silicate, aluminium hydroxide, aluminium phosphate, aluminium carbonate, dihydroxialuminium sodiumcarbonate, dihydroxialuminium aminoacetate or a mixture thereof.

4. A pharmaceutical composition according to claims 2 or 3 wherein said calcium salt is calcium carbonate and said aluminium salt is sodic aluminium silicate.

5. A pharmaceutical composition according to claim 1 wherein the lactic acid bacteria used are chosen among: streptoccus thermophilus, lactobacillus bulgaricus,-helveticus, -lactis, -acidophilus, -casei, -plantarum,-paracasei, bifidobacterium breve, -bifidum, -longum,-adolescentis, -infantis,.

6. A pharmaceutical composition according to claim 5 wherein a mixture is used of the following lactic acid bacteria: bifidobacterium longum, streptoccus thermophilus, lactobacillus bulgaricus, lactobacillus lactis, lactobacillus acidophilus, lactobacillus casei.

7. A pharmaceutical composition according to claim 1 wherein not adsorbable carbohydrates are added chosen between: lactulose, lactitol, sorbitol, mannitol, xilitol, maltitle, isomalte, raffinose, fruit oligosaccharides, inuline.

8. A pharmaceutical composition according to claim 1 wherein vitamins of group B are added chosen among: vit.B1, vit.B2, vit.B6, vit.B12, pantothenic acid.

9. A pharmaceutical composition according to the previous claims, wherein calcium carbonate, sodic aluminium silicate and the lactic acid bacteria are mixed according to the following general weight proportion:
- Calcium carbonate 500-2500mg
- Sodic aluminium silicate 500-4500mg
- Bifidobacterium longum 1-100 billion of units
- Lactobacillus lactis 1-100 billion of units
- Lactobacillus bulgaricus 1-300 billion of units
- Lactobacillus acidophilus 1-300 billion of units
- Lactobacillus casei 1-300 billion of units
- Streptococcus thermophilus 1-300 billion of units

10. A pharmaceutical composition according to claims from 1 to 8 **characterised** as follows:
- Calcium carbonate 500-2500mg
- Sodic aluminium silicate 500-4500mg
- Bifidobacterium bifidum 1-50 billion of units
- Lactobacillus paracasei 1-50 billion of units

## Patentansprüche

1. Lebende Milchsäurebakterien enthaltende pharmazeutische Zusammensetzung zur human- und tiermedizinischen Verwendung zur Regeneration der Darmflora bei Diarrhoe oder dyspeptischem Syndrom (Colitis, Diarrhoe, unspezifische Enteritis), für die Prophylaxe und Behandlung von Darmflorastörung nach der Verwendung von Antibiotika, für Digestionskrankheiten des künstlich ernährten Neugeborenen und für Digestionskrankheiten bei landwirtschaftlichen Nutztieren und bei Hobbytieren, **dadurch gekennzeichnet, daß** sie ein Calciumsalz und ein Aluminiumsalz als Zusatzstoffe enthält.

2. Pharmazeutische Zusammensetzung nach Anspruch 1, bei der die verwendeten Calciumsalze aus der Gruppe Calciumcarbonat, zweibasiges Calciumphosphat, dreibasiges Calciumphosphat oder einer Mischung davon stammen.

3. Pharmazeutische Zusammensetzung nach Anspruch 1, wobei die verwendeten Aluminiumsalze aus der Gruppe Natriumaluminiumsilikat, Calciumaluminiumsilikat, Aluminiummagnesiumsilikat, Aluminiumhydroxid, Aluminiumphosphat, Aluminiumcarbonat, Dihydroxyaluminiumnatriumcarbonat, Dihydroxyaluminiumaminoacetat oder einer Mischung davon stammen.

4. Pharmazeutische Zusammensetzung nach Anspruch 2 oder 3, wobei es sich bei dem Calciumsalz um Calciumcarbonat und bei dem Aluminiumsalz um Natriumaluminiumsilikat handelt.

5. Pharmazeutische Zusammensetzung nach Anspruch 1, wobei die verwendeten Milchsäurebakterien aus der Gruppe Streptococcus thermophilus, Lactobacillus bulgaricus, L. helveticus, L.lactis, L. acidophilus, L. casei, L. plantarum, L. paracasei, Bifidobacterium breve, B. bifidum, B. longum, B. adolescentis, B. infantis stammen.

6. Pharmazeutische Zusammensetzung nach Anspruch 5, bei der eine Mischung der folgenden Milchsäurebakterien verwendet wird: Bifidobacterium longum, Streptococcus thermophilus, Lactobacillus bulgaricus, Lactobacillus lactis, Lactobacillus acidophilus, Lactobacillus casei.

7. Pharmazeutische Zusammensetzung nach Anspruch 1 mit einem Zusatz von nicht adsorbierbaren Kohlehydraten aus der Gruppe Lactulose, Lactit, Sorbit, Mannit, Xylit, Maltit, Isomaltit, Raffinose, Frucht-Oligosaccharide, Inulin.

8. Pharmazeutische Zusammensetzung nach Anspruch 1 mit einem Zusatz von B-Vitaminen aus der Gruppe Vitamin B1, Vitamin B2, Vitamin B6, Vitamin B12, Pantothensaure.

9. Pharmazeutische Zusammensetzung nach den vorhergehenden Ansprüchen, bei der Calciumcarbonat, Natriumaluminiumsilikat und die Milchsäurebakterien im folgenden allgemeinen Gewichtsverhältnis gemischt werden:
- Calciumcarbonat 500-2500 mg
- Natriumaluminiumsilikat 500-4500 mg
- Bifidobacterium longum 1-100 Mrd. Einheiten
- Lactobacillus lactis 1-100 Mrd. Einheiten
- Lactobacillus bulgaricus 1-300 Mrd. Einheiten
- Lactobacillus acidophilus 1-300 Mrd. Einheiten
- Lactobacillus casei 1-300 Mrd. Einheiten
- Streptococcus thermophilus 1-300 Mrd. Einheiten

10. Pharmazeutische Zusammensetzung nach den Ansprüchen 1 bis 8, **gekennzeichnet durch**
- Calciumcarbonat 500-2500 mg
- Natriumaluminiumsilikat 500-4500 mg
- Bifidobacterium bifidum 1-50 Mrd. Einheiten
- Lactobacillus paracasei 1-50 Mrd. Einheiten

## Revendications

1. Composition pharmaceutique à usage médical et vétérinaire pour la régénération de la flore intestinale dans les diarrhées ou dans le syndrome dyspeptique (colite, diarrhée, entérite non spécifique), pour la prophylaxie et le traitement des désordres du microbisme intestinal après l'utilisation d'antibiotiques, pour les maladies digestives du nouveau-né nourri artificiellement, pour les maladies digestives des animaux de ferme et de compagnie, contenant des bactéries productrices d'acide lactique vivantes et **caractérisée en ce qu'**elle renferme un sel de calcium et un sel d'aluminium qui ont été ajoutés.

2. Composition pharmaceutique selon la revendication 1, dans laquelle les sels de calcium utilisés sont choisis parmi : le carbonate de calcium, le phosphate bibasique de calcium, le diphosphate tricalcique ou un mélange de ces sels.

3. Composition pharmaceutique selon la revendication 1, dans laquelle les sels d'aluminium utilisés sont choisis parmi : le silicate sodique d'aluminium, le silicate calcique d'aluminium, le silicate de magnésium et d'aluminium, l'hydroxyde d'aluminium, le phosphate d'aluminium, le carbonate d'aluminium, le carbonate sodique de dihydroxyaluminium, l'aminoacétate de dihydroxyaluminium ou un mélange de ces sels.

4. Composition pharmaceutique selon la revendication 2 ou 3, dans laquelle ledit sel de calcium est du carbonate de calcium et ledit sel d'aluminium est du silicate sodique d'aluminium.

5. Composition pharmaceutique selon la revendication 1, dans laquelle les bactéries productrices d'acide lactique utilisées sont choisies parmi : Streptococcus thermophilus, Lactobacillus bulgaricus, -helveticus, -lactis, -acidophilus, -casei, -plantarum, -paracasei, Bifidobacterium breve, -bifidum, -longum, -adolescentis, -infantis.

6. Composition pharmaceutique selon la revendication 5, dans laquelle un mélange des bactéries productrices d'acide lactique suivantes est utilisé : Bifidobacterium longum, Streptococcus thermophilus, Lactobacillus bulgaricus, Lactobacillus lactis, Lactobacillus acidophilus, Lactobacillus casei.

7. Composition pharmaceutique selon la revendication 1, dans laquelle les hydrates de carbone non absorbables ajoutés sont choisis parmi : le lactulose, le lactitol, le sorbitol, le mannitol, le xylitol, le maltol, l'isomaltol, le raffinose, des oligosaccharides de fruit, l'inuline.

8. Composition pharmaceutique selon la revendication 1, dans laquelle les vitamines du groupe B ajoutées sont choisies parmi : la vitamine B1, la vitamine B2, la vitamine B6, la vitamine B12, l'acide pantothénique.

9. Composition pharmaceutique selon les revendications précédentes, dans laquelle du carbonate de calcium, du silicate sodique d'aluminium et les bactéries productrices d'acide lactique sont mélangés selon la proportion générale en poids suivante :
- Carbonate de calcium 500 à 2500 mg
- Silicate sodique d'aluminium 500 à 4500 mg
- Bifidobacterium longum 1 à 100 milliards d'unités
- Lactobacillus lactis 1 à 100 milliards d'unités
- Lactobacillus bulgaricus 1 à 300 milliards d'unités
- Lactobacillus acidophilus 1 à 300 milliards d'unités
- Lactobacillus casei 1 à 300 milliards d'unités
- Streptococcus thermophilus 1 à 300 milliards d'unités

10. Composition pharmaceutique selon les revendications 1 à 8 et **caractérisée** comme suit :
- Carbonate de calcium 500 à 2500 mg
- Silicate sodique d'aluminium 500 à 4500 mg
- Bifidobacterium bifidum 1 à 50 milliards d'unités
- Lactobacillus paracasei 1 à 50 milliards d'unités
